# EUROPEAN PATENT APPLICATION

(11) **EP 4 162 957 A1**
(43) Date of publication of application: **12.04.2023**
(21) Application number: 21821626.5
(22) Date of filing: 09.06.2021
(51) Int. Cl.: A61L 2/18, C02F 1/50, C02F 1/76, C02F 1/78, E03C 1/126

(54) **INFECTIOUS DRAINAGE TREATMENT SYSTEM**

(30) Priority: 09.06.2020 JP 2020100441
(71) Applicant: Mec Co., Ltd., Tokyo 104-0052 (JP)
(72) Inventor: UEDA, Takao, Tokyo 104-0052 (JP); SAKAI, Yozo, Tokyo 104-0052 (JP)
(74) Representative: TBK
(86) International application number: PCT/JP2021/022007
(87) International publication number: WO 2021/251444

(57) **Abstract**

A drainage pipe disinfection apparatus and a drainage (wastewater) treatment system capable of effectively sterilizing and disinfecting drainage piping of sanitary equipment is provided. The disinfection apparatus for drainage piping of sanitary equipment comprises a cleansing tank, a toilet bowl which serves as the sanitary equipment, and a drainage pipe connected to the toilet bowl, wherein when the sanitary equipment is used, a fixed amount of cleansing water is supplied to the toilet bowl from the cleansing tank, and the cleansing water after cleansing the toilet bowl is drained from the drainage pipe; wherein the disinfection apparatus for drainage piping of sanitary equipment further comprises: replenishment piping configured to replenish, from a water supply system, a fixed amount of water corresponding to the amount of cleansing water supplied from the cleansing tank to the toilet bowl; a sensor configured to sense the supply of water to the cleansing tank; a chemical liquid tank configured to store a fixed concentration of chlorine dioxide; a chemical liquid pump configured to supply a fixed amount of chemical liquid from the chemical liquid tank to the cleansing tank; and a control unit configured to operate the chemical liquid pump by means of a water supply sensing signal from the sensor to supply a fixed amount of chlorine dioxide to the cleansing tank, thereby maintaining the concentration of chlorine dioxide in the cleansing water within the cleansing tank at a fixed concentration.

## Description

### TECHNICAL FIELD

The present invention relates, for example, to an infectious drainage (wastewater) treatment system equipped with drainage pipes for sanitary equipment such as toilets, wash basins, waste sinks, etc., in hospitals, nursing care facilities, etc., where a mass infection is likely to occur, i.e., where wastewater containing pathogens or viruses (hereinafter referred to as infectious wastewater) may flow in cases where an infection occurs.

### BACKGROUND OF THE INVENTION

Since the beginning of the year, coronavirus infections have spread globally, and clusters (mass infections) in closed spaces such as hospitals, elderly care facilities, etc., have become a problem. In order to prevent clusters of pathogens and viruses in various facilities such as hospitals, thorough sterilization and disinfection of the facilities has been carried out, and it has been pointed out that a secondary infection from drainage pipes in the facilities is one of the causes of the clusters.

For example, the feces of a patient isolated in an infectious disease room contains viruses and pathogens, which not only contaminate a sewage system but also contaminate piping through which the feces flow. In addition, pathogens and viruses also adhere to the floors of toilets, bathrooms, wash basins, etc., and the wastewater therefrom also contaminates piping, and the wastewater from the sinks for disposing of waste also contaminates the piping. Pathogens and viruses have been demonstrated to form biofilms and lurk on the inner surfaces of the drainage pipes.

The drainage pipes do not always have wastewater flowing through them, and the biofilms dry up, so that pathogens and viruses drift into the air in the drainage pipes. A drainage pipe is usually equipped with a U-shaped trap that holds water to prevent odors of the wastewater from entering a room, but pathogens and viruses remain in the wastewater that stays in the trap, and biofilms are formed in a pipe line leading to the trap, causing pathogens and viruses to flow back into the room with air currents, contaminating the room. In particular, if the water in the trap evaporates, a large amount of viruses and pathogens floating in the pipe line downstream of the trap will flow into the room.

In view of such a fear of secondary infection from the infectious wastewater, the need for sterilization and disinfection of drainage pipes is recognized, but due to the complexity thereof such as, for example, the preparation of diluted chlorine dioxide water, discharging of the water by hand, etc., this is rarely implemented. In addition, even if such discharging is carried out, a sufficient effect cannot be obtained at present due to the timing of the discharging or the like.

The applicant has previously proposed a drainage treatment method and a treatment apparatus for infectious waste, as described in Patent Literature 1, but while the outflow thereof into the external environment can be prevented, no consideration has been given to the treatment in the piping to the treatment apparatus.

### Citation List

### Patent Literature

Patent Literature 1: Japanese Patent No. 3496094

### SUMMARY OF THE INVENTION

### Technical Problem

An object of the present invention is to provide a drainage pipe disinfection apparatus and a drainage (wastewater) treatment system capable of effectively sterilizing and disinfecting drainage piping of sanitary equipment.

### Solution to Problem

In order to achieve the above-mentioned object, a first invention is directed to an infectious drainage treatment system, which is characterized by comprising a disinfection apparatus for drainage piping of sanitary equipment, and a wastewater sterilization treatment apparatus arranged downstream of the drainage piping;
wherein the disinfection apparatus for drainage piping of sanitary equipment comprises a cleansing tank, a toilet bowl which serves as the sanitary equipment, and a drainage pipe connected to the toilet bowl, wherein when the sanitary equipment is used, a fixed amount of cleansing water is supplied to the toilet bowl from the cleansing tank, and the cleansing water after cleansing the toilet bowl is drained from the drainage pipe;
wherein the disinfection apparatus for drainage piping of sanitary equipment further comprises:
   replenishment piping configured to replenish, from a water supply system, a fixed amount of water corresponding to the amount of cleansing water supplied from the cleansing tank to the toilet bowl;
   a sensor configured to sense the supply of water to the cleansing tank;
   a chemical liquid tank configured to store a fixed concentration of chlorine dioxide;
   a chemical liquid pump configured to supply a fixed amount of chemical liquid from the chemical liquid tank to the cleansing tank; and
   a control unit configured to operate the chemical liquid pump by means of a water supply sensing signal from the sensor to supply a fixed amount of chlorine dioxide to the cleansing tank, thereby maintaining the concentration of chlorine dioxide in the cleansing water within the cleansing tank at a fixed concentration;
wherein the wastewater sterilization treatment apparatus is a treatment apparatus that performs treatment with chlorine dioxide and treatment with ozone on wastewater from the drainage pipe; and
wherein the wastewater sterilization treatment apparatus has a raw water tank for storing wastewater from the drainage pipe, and the drainage pipe is connected to a screen for removing foreign substances flowing into the raw water tank.

According to the first invention, when the toilet is in use, the toilet and the drainage pipe are disinfected and sterilized by the cleansing water containing chlorine dioxide of the fixed concentration supplied from the cleansing tank to the toilet, so that biofilms in the drainage pipe are removed.

In addition, when replenishment water is replenished to the cleansing tank, a fixed amount of stabilized chlorine dioxide is replenished from the chemical liquid tank, so that the concentration of chlorine dioxide in the cleansing water can always be maintained constant.

Further, it is possible to prevent infection in the facility, and at the same time, prevent the release of pathogens and viruses from the source of infection into the external environment. The chlorine dioxide, which is used for disinfection of the drainage pipe and is present in the wastewater, may be added in the wastewater sterilization treatment apparatus or may not be added, depending on the concentration thereof.

Furthermore, the screen requires maintenance on a regular basis, but if the screen is configured to receive wastewater from the drainage pipes, as in the present invention, the screen is exposed to the chlorine dioxide contained in the wastewater, so that the screen can be sterilized. As a result, the infection risk at the time of maintenance of the screen can be reduced. Moreover, the raw water tank is always filled with the fixed concentration of chlorine dioxide water, so that pathogens and viruses are inactivated.

A second invention is directed to an infectious drainage treatment system, which is characterized by comprising a disinfection apparatus for drainage piping of sanitary equipment, and a wastewater sterilization treatment apparatus arranged downstream of the drainage piping;
wherein the disinfection apparatus for drainage piping of sanitary equipment comprises sanitary equipment with a water faucet for water supply, and a drainage pipe connected to the sanitary equipment;
wherein the disinfection apparatus for drainage piping of sanitary equipment, in which wastewater is drained from the drainage pipe, further comprises:
   a cleansing tank configured to store cleansing water for cleansing the sanitary equipment;
   a discharge port for the cleansing water provided in the sanitary equipment separately from the water faucet;
   cleansing water piping connecting between the cleansing tank and the discharge port;
   an electric valve provided in the cleansing water piping to open and close a flow passage in the cleansing water piping;
   replenishment piping configured to replenish, from a water supply system, a fixed amount of water corresponding to the amount of cleansing water supplied from the cleansing tank to the sanitary equipment;
   a sensor configured to sense the supply of water to the cleansing tank;
   a chemical liquid tank configured to store a fixed concentration of chlorine dioxide;
   a chemical liquid pump configured to supply a fixed amount of chemical liquid from the chemical liquid tank to the cleansing tank; and
   a control unit configured to operate the electric valve at a fixed cycle to supply a fixed amount of cleansing water to the sanitary equipment through the discharge port, and to operate the chemical liquid pump to supply a fixed amount of chlorine dioxide to the cleansing tank by means of a water supply sensing signal from the sensor to maintain the concentration of chlorine dioxide in the cleansing water within the cleansing tank at a fixed concentration;
   wherein the wastewater sterilization treatment apparatus is a treatment apparatus that performs treatment with chlorine dioxide and treatment with ozone on wastewater from the drainage pipe; and
   wherein the wastewater sterilization treatment apparatus has a raw water tank for storing wastewater from the drainage pipe, and the drainage pipe is connected to a screen for removing foreign substances flowing into the raw water tank.

According to this second invention, cleansing water is automatically supplied to the sanitary equipment through the discharge port at a fixed cycle to sterilize and disinfect the sanitation equipment and the drainage pipe leading to the sanitary equipment, so that biofilms in the drainage pipe are removed.

In addition, when a fixed amount of replenishment water is replenished, a fixed amount of stabilized chlorine dioxide is replenished from the chemical liquid tank, so that the concentration of chlorine dioxide in the cleansing water can always be maintained constant.

Further, it is possible to prevent infection in the facility, and at the same time, prevent the release of pathogens and viruses from the source of infection into the external environment. The chlorine dioxide, which is used for disinfection of the drainage pipe and is present in the wastewater, may be added in the wastewater sterilization treatment apparatus or may not be added, depending on the concentration thereof.

Furthermore, the screen requires maintenance on a regular basis, but if the screen is configured to receive wastewater from the drainage pipes, as in the present invention, the screen is exposed to the chlorine dioxide contained in the wastewater, so that the screen can be sterilized. As a result, the infection risk at the time of maintenance of the screen can be reduced. Moreover, the raw water tank is always filled with the fixed concentration of chlorine dioxide water, so that pathogens and viruses are inactivated.

In addition, an infectious drainage treatment system according to a third invention is characterized by comprising a disinfection apparatus for first drainage piping of first sanitary equipment, a disinfection apparatus for second drainage piping of second sanitary equipment, and a wastewater sterilization treatment apparatus arranged downstream of the first drainage piping and the second drainage piping;
wherein the disinfection apparatus for first drainage piping of first sanitary equipment comprises a first cleansing tank, a toilet bowl which serves as the first sanitary equipment, and a first drainage pipe connected to the toilet bowl, wherein when the toilet bowl is used, a fixed amount of cleansing water is supplied to the toilet bowl from the first cleansing tank, and the cleansing water after cleansing the toilet bowl is drained from the first drainage pipe;
wherein the disinfection apparatus for drainage piping of first sanitary equipment further comprises:
   replenishment piping configured to replenish, from a water supply system, a fixed amount of water corresponding to the amount of cleansing water supplied from the first cleansing tank to the toilet bowl;
   a sensor configured to sense the supply of water to the first cleansing tank;
   a chemical liquid tank configured to store a fixed concentration of chlorine dioxide;
   a chemical liquid pump configured to supply a fixed amount of chemical liquid from the chemical liquid tank to the first cleansing tank; and
   a control unit configured to operate the chemical liquid pump by means of a water supply sensing signal from the sensor to supply a fixed amount of chlorine dioxide to the first cleansing tank, thereby maintaining the concentration of chlorine dioxide in the cleansing water within the first cleansing tank at a fixed concentration;
   wherein the disinfection apparatus for drainage piping of second sanitary equipment comprises second sanitary equipment with a water faucet for water supply, and a second drainage pipe connected to the second sanitary equipment;
wherein the disinfection apparatus for drainage piping of sanitary equipment, in which wastewater is drained from the second drainage pipe, further comprises:
   a second cleansing tank configured to store cleansing water for cleansing the second sanitary equipment;
   a discharge port for the cleansing water provided in the second sanitary equipment separately from the water faucet;
   cleansing water piping connecting between the cleansing tank and the discharge port;
   an electric valve provided in the cleansing water piping to open and close a flow passage in the cleansing water piping;
   replenishment piping configured to replenish, from a water supply system, a fixed amount of water corresponding to the amount of cleansing water supplied from the cleansing tank to the second sanitary equipment;
   a sensor configured to sense the supply of water to the second cleansing tank;
   a chemical liquid tank configured to store a fixed concentration of chlorine dioxide;
   a chemical liquid pump configured to supply a fixed amount of chemical liquid from the chemical liquid tank to the cleansing tank; and
   a control unit configured to operate the electric valve at a fixed cycle to supply a fixed amount of cleansing water to the second sanitary equipment through the discharge port, and to operate the chemical liquid pump to supply a fixed amount of chlorine dioxide to the second cleansing tank by means of a water supply sensing signal from the sensor to maintain the concentration of chlorine dioxide in the cleansing water within the second cleansing tank at a fixed concentration;
   wherein the wastewater sterilization treatment apparatus is a treatment apparatus that performs treatment with chlorine dioxide and treatment with ozone on wastewater from the first drainage pipe and the second drainage pipe; and
   wherein the wastewater sterilization treatment apparatus has a raw water tank for storing wastewater from the first drainage pipe and the second drainage pipe, and the drainage pipes are connected to a screen for removing foreign substances flowing into the raw water tank.

According to the third invention, when the toilet is in use, the toilet and the first drainage pipe are disinfected and sterilized by the cleansing water containing chlorine dioxide of the fixed concentration supplied from the first cleansing tank to the toilet, so that biofilms in the first drainage pipe are removed.

In addition, cleansing water is automatically supplied from the discharge port to the second sanitary equipment at a fixed cycle, so that the second sanitary equipment and the second drainage pipe leading to the second sanitary equipment are disinfected and sterilized, thereby removing biofilms in the second drainage pipe.

Moreover, when a fixed amount of replenishment water is replenished, a fixed amount of stabilized chlorine dioxide is replenished from the chemical liquid tank, so that the concentration of chlorine dioxide in the cleansing water can always be maintained constant.

Further, it is possible to prevent infection in the facility, and at the same time, prevent the release of pathogens and viruses from the source of infection into the external environment. The chlorine dioxide, which is used for disinfection of the first drainage pipe and the second drainage pipe and is present in the wastewater, may be added in the wastewater sterilization treatment apparatus or may not be added, depending on the concentration thereof.

Furthermore, the screen requires maintenance on a regular basis, but if the screen is configured to receive wastewater from the drainage pipes, as in the present invention, the screen is exposed to the chlorine dioxide contained in the wastewater, so that the screen can be sterilized. As a result, the infection risk at the time of maintenance of the screen can be reduced. Moreover, the raw water tank is always filled with the fixed concentration of chlorine dioxide water, so that pathogens and viruses are inactivated.

The first through third inventions can be configured as follows.

The drainage pipe in the first and second inventions is a dedicated drainage pipe.

Also, the first drainage pipe and the second drainage pipe in the third invention are also dedicated drainage pipes.

If the drainage pipes in the first invention and the second invention, and the first drainage pipe and the second drainage pipe in the third invention are connected to drainage pipes other than the infectious system, there is a possibility that secondary contamination may be caused in the rooms of departments other than the infectious system through the drainage pipes other than the infectious system. By providing the dedicated drainage pipes in this way, it is possible to prevent secondary contamination.

A plurality of raw water tanks may be provided, and the raw water tank into which wastewater flows may be any one of them, and a means may be provided for switching to another raw water tank when an amount of wastewater stored in the raw water tank in use exceeds an acceptable amount.

If a plurality of raw water tanks are provided, the facility can be reduced in size, and maintenance, such as cleaning of the raw water tanks or the like, can be performed without stopping the treatment facility by stopping the raw water tank to be maintained while keeping the other raw water tanks in operation.

In addition, the wastewater sterilization treatment apparatus can be configured such that a fixed amount of wastewater is supplied from the raw water tanks to a reaction tank for treatment, wherein in cases where wastewater is supplied from a raw water tank into which wastewater is flowing, the supply of wastewater from the raw water tank to the reaction tank is continued until the amount of wastewater stored in the raw water tank becomes equal to or less than a specified amount, even after the amount of wastewater stored therein exceeds the acceptable amount to switch the supply from the raw water tank to another raw water tank, whereas when the amount of stored wastewater becomes equal to or less than the specified amount, the supply is switched to the supply from the other raw water tank.

In this way, the supply from the plurality of raw water tanks to the reaction tank can be smoothly performed.

The reaction tank of the wastewater sterilization treatment apparatus is provided with a circulation path that serves to discharge treated wastewater in the reaction tank to the outside and then return it thereto, and a fine bubble supplying means is provided for supplying ozone to the circulation path as fine bubbles.

The term "fine bubbles" as used in the present description includes microbubbles of 1 µm or more and 100 µm or less and nanobubbles of less than 1 µm, which are collectively referred to as fine bubbles of 100 µm or less. The upper limit thereof is not strictly defined as 100 µm, but fine bubbles are referred to as such in the present description in the sense that the bubbles are smaller than about 100 µm.

By using the fine bubbles, the area of the gas-liquid interface of the ozone fine bubbles dispersed in the wastewater dramatically increases, so that the chlorine dioxide dissolved therein is also activated as bubbles, increasing the probability and degree to which pathogens such as spore-forming bacteria or the like represented by anthrax, other bacteria, viruses, residual drugs, various toxic substances, etc., present in the wastewater are exposed to the ozone. As a result, decomposition treatment of these substances is dramatically accelerated, and infectious waste can be treated efficiently at a lower cost without increasing the size of the treatment facility.

Here, note that chlorine dioxide may also be supplied to the wastewater as fine bubbles through a fine bubble generating device, or a fine bubble generating device may also be arranged in the reaction tank to generate dissolved chlorine dioxide as fine bubbles, so that the fine bubbles of the chlorine dioxide, in combination with the fine bubbles of ozone, can further promote decomposition treatment.

Further, a fourth invention is directed to an infectious drainage treatment system, which is characterized by comprising a wastewater sterilization treatment apparatus arranged downstream of drainage piping, wherein the wastewater sterilization treatment apparatus performs treatment with chlorine dioxide and treatment with ozone on wastewater from the drainage piping, and
wherein a reaction tank of the wastewater sterilization treatment apparatus is provided with a circulation path that serves to discharge treated wastewater in the reaction tank to the outside and then return it thereto, and a fine bubble supplying means is provided for supplying ozone to the circulation path as fine bubbles.

The chlorine dioxide may also be supplied to the wastewater as microbubbles through a fine bubble generating device.

### Advantageous Effects of Invention

According to the present invention, cleansing and sterilization of drainage pipes by chlorine dioxide is automatically performed, so that a secondary infection in facilities such as hospitals, nursing care facilities, etc., in which a mass infection is likely to occur, can be significantly reduced.

In addition, in cases where an infectious waste treatment apparatus is used in combination, it is possible to prevent infection in facilities and at the same time prevent pathogens and viruses in a source of infection from being released into the external environment.

### BRIEF DESCRIPTION OF THE DRAWINGS

FIG. 1 is a schematic configuration diagram of a disinfection apparatus for drainage piping of sanitary equipment applied to an infectious drainage treatment system according to a first embodiment of the present invention.
FIG. 2 is a schematic configuration diagram of a disinfection apparatus for drainage piping of sanitary equipment applied to an infectious drainage treatment system according to a second embodiment of the present invention.
FIG. 3 is a schematic configuration diagram of an infectious drainage treatment system in which a disinfection apparatus for drainage piping of sanitary equipment and a wastewater sterilization treatment apparatus are combined with each other, according to the present invention.

### EMBODIMENTS FOR CARRYING OUT THE INVENTION

Hereinafter, the present invention will be described on the basis of embodiments illustrated in the drawings.

### First Embodiment

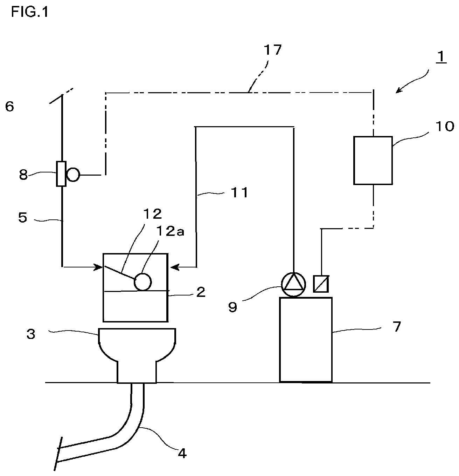

FIG. 1 is a schematic configuration diagram of a disinfection apparatus for drainage piping of sanitary equipment according to a first embodiment applied to an infectious drainage treatment system of the present invention.

This disinfection apparatus 1 is applied to drainage piping of a toilet bowl 3, which is sanitary equipment in hospitals or other facilities used by people infected or potentially infected with pathogens or viruses.

That is, the disinfection apparatus 1 includes a cleansing tank 2, the toilet bowl 3 which serves as sanitary equipment, and a drainage pipe 4 connected to the toilet bowl 3, wherein when the toilet bowl 3 is used, a fixed amount of cleansing water is supplied to the toilet bowl 3 from the cleansing tank 2, and the cleansing water after cleansing the toilet bowl 3 is drained from the drainage pipe 4. The cleansing water stored in the cleansing tank 2 contains a fixed concentration of chlorine dioxide, which washes or cleanses the toilet bowl 3 and sterilizes the drainage pipe 4.

The disinfection apparatus 1 further includes replenishment water piping 5 for replenishing the cleansing tank 2 with water, a sensor 8 for sensing the supply of water to the cleansing tank 2, a chemical liquid tank 7 for storing chlorine dioxide, a chemical liquid pump 9, and a control unit 10 for controlling the driving of the chemical liquid pump 9.

The cleansing tank 2 is a so-called low tank, and is arranged adjacent to the toilet bowl 3. A float valve 12 is provided in the cleansing tank 2. The float valve 12 is configured such that when a float 12a floating on a liquid surface becomes lower than a predetermined height, a valve member located at the root is opened to start supplying water, and when the liquid surface or level reaches a predetermined position, the valve member is mechanically closed to stop the water supply.

The replenishment water piping 5 connects a water supply system 6 and the cleansing tank 2 with each other, and replenishes, from the water supply system 6, a fixed amount of water corresponding to the amount of cleansing water supplied to the toilet bowl 3.

The sensor 8 serves to sense the flow of water flowing through the replenishment water piping 5, and sends a sensing signal to the control unit 10, for example, when the flow exceeds a predetermined value. The sensor 8 may have a function of sensing the start and stop of the water supply to the cleansing tank (low tank) 2, and for example, a flow velocity sensor or a flow rate sensor can be used, wherein when the flow velocity or the flow rate reaches a level equal to or higher than the predetermined value, the sensor 8 sends a sensing signal.

The chemical liquid tank 7 is a dedicated tank, which is arranged at a distance from the side of the toilet bowl 3, and is connected to the cleansing tank 2 through a chemical liquid piping 11. The chemical liquid tank 7 contains chlorine dioxide. The chlorine dioxide is used in the form of an aqueous solution, particularly in the form of stabilized chlorine dioxide which is stabilized by dissolving chlorine dioxide in an alkaline solution.

The chemical liquid pump 9 is attached to the chemical liquid tank 7, so that the stabilized chlorine dioxide in the chemical liquid tank 7 is drawn up and pressure-fed to the cleansing tank. As the chemical liquid pump 9, there is used an electrically operated pump.

The sensor 8 and the chemical liquid pump 9 are connected to a control unit 10 through control wiring 17.

The control unit 10 is configured to operate the chemical liquid pump 9 by means of a water supply sensing signal from the sensor 8, so that a fixed amount of chlorine dioxide is supplied to the cleansing tank 2, thereby maintaining the concentration of chlorine dioxide in the cleansing water within the cleansing tank 2 at a fixed concentration.

### Description of Operation

When the cleansing water is caused to flow from the cleansing tank 2 to the toilet bowl 3, the liquid level in the cleansing tank 2 drops, and, for example, the float valve 12 is opened, so that replenishment water is supplied from the water supply system 6 into the cleansing tank 2 through the replenishment water piping 5. The sensor 8 senses this replenished water flow, and transmits a sensing signal to the control unit 10. When the sensing signal for the water flow is input, the control unit 10 transmits a drive signal for the chemical liquid pump 9 to drive the chemical liquid pump 9 for a fixed period of time, so that a fixed amount of water is supplied to the cleansing tank 2 through the chemical liquid piping 11.

The amount of water supplied from the water supply system 6 to the cleansing tank 2 has been determined in advance as a fixed amount, and the amount of chlorine dioxide supplied to the cleansing tank 2 through the chemical liquid piping 11 has also been determined in advance as a fixed amount, as a result of which the concentration of the chlorine dioxide in the cleansing tank 2 is kept at a constant level.

Therefore, in the next use and cleansing operation of the toilet bowl 3, the cleansing water accumulated in the cleansing tank 2, i.e., the diluted chlorine dioxide solution (disinfectant), flows from the toilet bowl 3 to the drainage pipe 4, so that the inner wall of the drainage pipe 4 is disinfected and sterilized.

The replenishment water and the chlorine dioxide in the cleansing tank 2 are agitated, so that the cleansing water therein reaches a specified concentration of the chlorine dioxide according to the activation time of the chemical liquid pump 9 set by the control unit 10.

According to the first embodiment, when the toilet bowl 3 is in use, the toilet bowl 3 and the drainage pipe 4 are disinfected and sterilized by the cleansing water containing the fixed concentration of chlorine dioxide supplied from the cleansing tank 2 to the toilet bowl 3, so that biofilms in the drainage pipe 4 are removed.

In addition, when replenishment water is replenished to the cleansing tank 2, a fixed amount of stabilized chlorine dioxide is replenished from the chemical liquid tank 7, so that the concentration of chlorine dioxide in the cleansing water can always be maintained constant.

### Second Embodiment

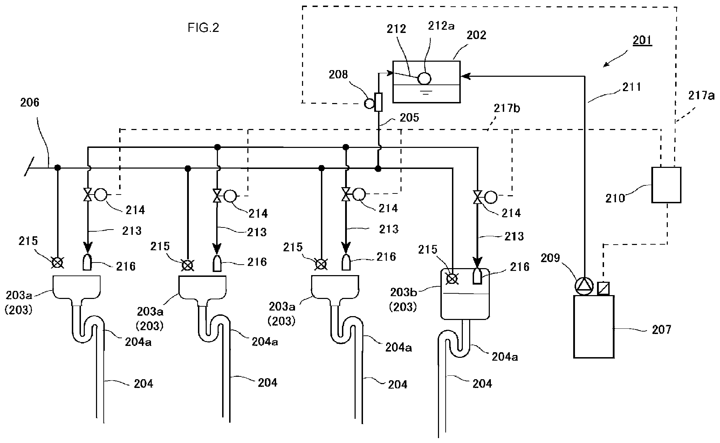

FIG. 2 is a schematic configuration diagram of a disinfection apparatus for drainage piping of sanitary equipment applied to an infectious drainage treatment system of a second embodiment of the present invention.

The disinfection apparatus 201 of the second embodiment is an example of sterilizing and disinfecting drainage pipes 204 of hand wash basins 203a and a waste sink 203b. The drainage pipes 204 are provided with traps 204a, respectively. In the illustrated example, the hand wash basins 203a are arranged at a plurality of locations and the waste sink 203b is arranged at one location, but as to the number of arrangements, a hand wash basin 203a may be arranged at one location, and waste sinks 203b may be arranged at a plurality of locations, or at any number of locations. In the following description, they are collectively referred to as sanitary equipment 203 unless otherwise required.

That is, the disinfection apparatus 201 includes a single cleansing tank 202, water supply or water faucets 215 provided respectively in sanitary equipment 203, discharge ports 216 provided separately from the water faucets 215, cleansing water piping 213 connecting between the cleansing tank 202 and the discharge ports 216, and electric valves 214 arranged in the cleansing water piping 213 to open and close the flow passages of the cleansing water piping 213.

The water faucets 215 respectively provided in the sanitary equipment 203 are connected to the main water supply system 206 through branch piping 206a, and water or hot water is supplied through the main water supply system 206 and the branch piping 206a.

In addition, provision is further made for a replenishment piping 205 for replenishing a fixed amount of water corresponding to the amount of the cleansing water supplied from the cleansing tank 202 to the sanitary equipment 203 from the main water supply system 206, a sensor 208 for sensing the water supply to the cleansing tank 202, a chemical liquid tank 207 for storing a fixed concentration of chlorine dioxide, a chemical liquid pump 209 for supplying a fixed amount of chemical liquid from the chemical liquid tank 207 to the cleansing tank 202, and a control unit 210 for controlling the electric valves 214 and the chemical liquid pump 209.

The cleansing tank 202 is a so-called high tank, which is arranged at a high place above the sanitary equipment 203 and is connected to the discharge ports 216 from common piping 213a via a branch piping 213b and the electric valve 214 is arranged in each branch piping 213b.

A float valve 212 is arranged in the cleansing tank 202, as in the cleansing tank 202 of the first embodiment. The float valve 212 is configured such that when a float 212a floating on a liquid surface becomes lower than a predetermined height, a valve member located at the root is opened to start supplying water, and when the liquid surface or level reaches a predetermined position, the valve member is mechanically closed to stop the water supply.

The replenishment piping 205 connects the water supply system 206 and the cleansing tank 202 with each other, and replenishes, from the water supply system 206, a fixed amount of water corresponding to the amount of cleansing water supplied to the sanitary equipment 203.

The sensor 208 senses the flow of water flowing through the replenishment piping 205, and, for example, transmits a sensing signal to the control unit 210 upon sensing the water flow. The sensor 208 has a function of sensing the start and stop of the supply of water to the cleansing tank (high tank) 202, and for example, a flow velocity sensor or a flow rate sensor can be used, wherein when the flow velocity or the flow rate reaches a level equal to or higher than a predetermined value, the sensor 208 sends a sensing signal.

The chemical liquid tank 207 is a dedicated tank, which is arranged at a distance from the side of the sanitary equipment 203, and is connected to the cleansing tank 202 through chemical liquid piping 211. The chemical liquid tank 207 stores stabilized chlorine dioxide, and the stabilized chlorine dioxide is replenished periodically or when it runs low.

The chemical liquid tank 207, which is the dedicated tank, is arranged away from the side of the sanitary equipment 203, and is connected to the cleansing tank 202 through the chemical liquid piping 211. The chlorine dioxide is stored in the chemical liquid tank 207 in the form of stabilized chlorine dioxide.

The chemical liquid pump 209 is attached to the chemical liquid tank 207, so that the stabilized chlorine dioxide in the chemical liquid tank 207 is drawn up and pressure-fed to the cleansing tank 202. As the chemical liquid pump 209, there is used an electrically operated pump.

The sensor 208, the chemical liquid pump 209 and the electric valves 214 are connected to the control unit 10 via control wiring 217.

The control unit 210 is configured to operate the electric valves 214 at a fixed cycle via the control wiring 217b to supply a fixed amount of cleansing water to the sanitary equipment through the discharge ports 216, and to operate the chemical liquid pump 209 to supply a fixed amount of chlorine dioxide to the cleansing tank 202 by means of a water supply sensing signal from the sensor 208 via the control wiring 217a, so that the concentration of chlorine dioxide in the cleansing water within the cleansing tank 202 is maintained at a fixed concentration.

### Operation

In each sanitary equipment 203, water is supplied from the water supply system 206 through each water faucet 215, and is used for hand washing and waste treatment, so that wastewater is discharged to the drainage pipe 204.

In this second embodiment, unlike the first embodiment, when each sanitary equipment 203 is used, instead of supplying cleansing water from the cleansing tank 202 to each sanitary equipment 203, each electric valve 214 is operated in a preset fixed cycle, so that cleansing water is supplied from each discharge port 216 to each sanitary installation 203. The sanitary equipment 203 and the drainage pipes 204 connected to the sanitary equipment 203 are disinfected and sterilized by the cleansing water thus supplied, and the biofilms of the drainage pipes 204 are removed. In the illustrated example, this cycle may be carried out simultaneously in three hand wash basins 203a and one waste sink 20b, or the cycle may be determined individually according to the frequency of use.

In addition, when a fixed amount of replenishment water is replenished into the cleansing tank 202, a fixed amount of stabilized chlorine dioxide is replenished from the chemical liquid tank 207, so that the concentration of stabilized chlorine dioxide in the cleansing water in the cleansing tank 202 can always be maintained constant.

### Third Embodiment

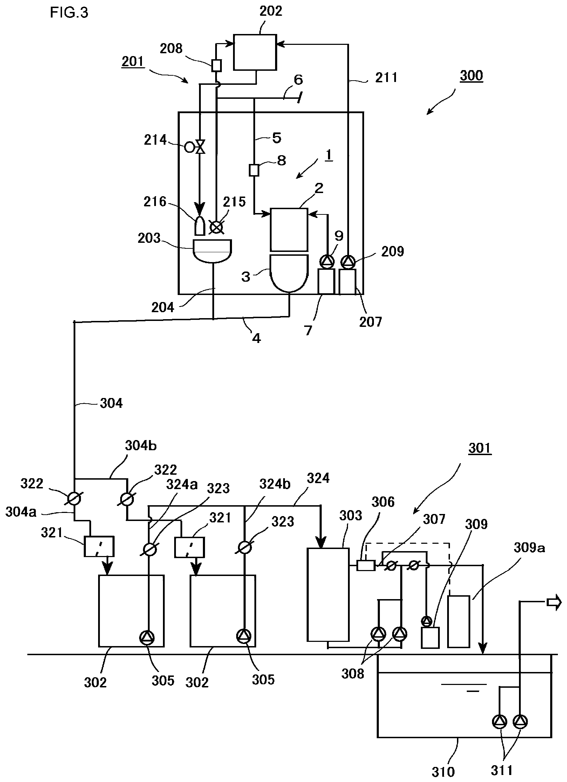

Next, a third embodiment will be described with reference to FIG. 3.

FIG. 3 is a schematic configuration diagram of an infectious drainage treatment system in which a disinfection apparatus for drainage piping and an infectious waste treatment apparatus as a wastewater sterilization treatment apparatus are combined with each other, according to the present invention.

The illustrated example shows an infectious hospital room. The wastewater discharged through drainage pipes 4, 204, 304 after an infected patient uses a toilet bowl 3 (first sanitary equipment in the third invention) or sanitary equipment 203 (second sanitary equipment in the third invention) needs to be safely managed from the beginning. An infectious drainage treatment system 300 of the third embodiment takes into consideration the safety of the entire wastewater, and is a system in which the disinfection apparatus 1 for the drainage pipe 4 (a first drainage pipe in the third invention) of the toilet bowl 3 of the first embodiment, the disinfection apparatus 201 for the drainage pipe 204 (a second drainage pipe in the third invention) of the sanitary equipment 203 such as hand wash basin of the second embodiment, and the infectious waste treatment apparatus 301 are combined with one another. The disinfection apparatus 1 for drainage piping of the first embodiment and the infectious waste treatment apparatus 301 may be combined with each other, or the disinfection apparatus 201 for drainage piping of the second embodiment and the infectious waste treatment apparatus 301 may be combined with each other.

The drainage pipe 4 of the toilet bowl 3 and the drainage pipe 204 of the hand wash basin 203 merge into a single drainage pipe 304, and downstream of this drainage pipe 304 is arranged an infectious waste treatment apparatus 301 for final detoxification of infectious wastewater, which is configured to discharge the wastewater treated to the outside environment.

The disinfection apparatus 1 for the drainage pipe 4 and the disinfection apparatus 201 for the drainage pipe 204 are the same as those shown in the first and second embodiments, and the same components are denoted by the same reference numerals, and descriptions thereof are omitted.

Here, the cleansing tank 2 corresponds to a first cleansing tank in the third invention, and the cleansing tank 202 corresponds to a second cleansing tank in the third invention.

The infectious waste treatment apparatus 301 includes raw water tanks 302, a reaction tank 303, a circulation path 307 for circulating the wastewater in the reaction tank 303, and wastewater supply devices 305 for supplying wastewater containing infectious waste to the reaction tank 303, a circulation device 308 for circulating the wastewater in the reaction tank 303 through the circulation path 307, an ozone supplying device 309a for supplying ozone to the circulation path 307, a chemical liquid injection device 309 for injecting chlorine dioxide into the circulation path 307, a discharge tank 310 for temporarily storing treated wastewater, and a discharge device 311 for discharging the treated wastewater from the discharge tank 310. The circulation path 307 is configured to discharge the treated wastewater in the reaction tank 303 to the outside and then return it to the reaction tank, and the circulation path 307 is provided with a fine bubble generating device 306 as a fine bubble supplying means that turns the ozone supplied from the ozone supplying device 309a into fine bubbles.

The fine bubble generating device 306 serves to narrow a part of the path or passage to make the flow velocity faster and the pressure lower by the venturi effect, thereby self-suctioning ozone gas from an intake port, so that a number of separated flows are generated in a portion where the passage is widened, thus making finer the bubbles of the ozone gas mixed in the wastewater by the shearing action of the separated flows. In this way, ozone is supplied into the wastewater as fine bubbles.

The raw water tanks 302 store the wastewater discharged from the drainage pipe 304, and are each provided with a screen 321 for removing foreign substances in the wastewater discharged from the drainage pipe 304.

Although not shown in particular, the infectious waste treatment apparatus 301 collectively treats or processes the infectious waste generated in autopsy rooms, operating rooms, etc., together with the wastewater from various sanitary equipment including the toilet bowl 3 and the hand wash basin 203. According to the Waste Disposal and Public Cleansing Law, infectious waste is classified into pathological waste (organs, tissues, etc.) generated by autopsy, surgery or the like, and infectious industrial waste containing blood, serum, plasma, and body fluids (including semen) generated by autopsy, surgery or the like, and the infectious waste referred to herein mainly falls under the infectious industrial waste. Of course, it is not limited to the legal definition, and can be applied to medical waste in general that needs to be sterilized. In the present embodiment, the infectious waste treatment apparatus 301 is used to illustrate, by way of example, the case where the treatment of infectious waste and the treatment of wastewater from sanitary equipment are performed by one treatment apparatus, but the present invention performs the treatment of wastewater flowing from drainage pipes in a drainage pipe disinfection apparatus, and it is directed to systems that can be widely applied not only to hospitals with such equipment as autopsy rooms, operating rooms, etc., but also to hospitals, nursing care facilities, etc., where a mass infection is likely to occur, and thus the treatment apparatus is not limited to the treatment combined with infectious waste.

Here, the treatment process of the infectious waste treatment apparatus 301 will be briefly described.

For wastewater treatment, a fixed amount of wastewater is supplied from the raw water tanks 302 to the reaction tank 303 to start treatment. When the treatment is started, the pump of the circulation device 308 causes the waste water to flow out from the outflow port of the reaction tank 303 into the circulation path, and the waste water flows into the reaction tank 303 from the return port. In the circulating wastewater, chlorine dioxide is supplied from the pump of the chemical liquid injection device 309 and ozone is supplied from the fine bubble generating device 306 as fine bubbles, whereas in the reaction tank 303, the sterilization and decomposition of pathogens and viruses as well as the decomposition of organic substances, etc., are performed, by the chlorine dioxide and the ozone. By using the fine bubbles, the area of the gas-liquid interface of the ozone fine bubbles dispersed in the wastewater dramatically increases, so that the chlorine dioxide dissolved therein is also activated as bubbles, increasing the probability and degree to which pathogens such as spore-forming bacteria or the like represented by anthrax, other bacteria, viruses, residual drugs, various toxic substances, etc., present in the wastewater are exposed to the ozone. As a result, decomposition treatment of these substances is dramatically accelerated, and infectious waste can be treated efficiently at a lower cost without increasing the size of the treatment facility.

Here, note that chlorine dioxide may also be supplied to the wastewater as fine bubbles through the fine bubble generating device, or a fine bubble generating device may also be arranged in the reaction tank to generate dissolved chlorine dioxide as fine bubbles, so that the fine bubbles of the chlorine dioxide, in combination with the fine bubbles of ozone, can further promote the decomposition treatment. In addition, the fine bubble generating device is not limited to the ejector type as described above, but various types can be applied depending on the equipment, such as a venturi type in which fine bubbles are generated by a venturi, a swirling flow type, a static mixer type, a microporous type, a pressurized dissolution type, an ultrasonic cavitation type, and the like.

Here, note that in the present embodiment, the chemical liquid is injected by the pump of the chemical liquid injection device 309 that injects the chlorine dioxide, but the chlorine dioxide is used for disinfecting the drainage pipe and exists in the wastewater stored in the raw water tanks 302, and hence, the amount thereof to be injected can be small. In addition, depending on the concentration of the chemical liquid, it may not be necessary to inject it from the chemical liquid injection device 309. In the present embodiment, a chemical liquid injection device 309 is provided because it also serves to treat infectious waste, but in cases where it is not also used to treat infectious waste, there may be an option of not providing a chemical liquid injection device 309 if the concentration of the chlorine dioxide in the raw water tanks is sufficient. Here, note that even if there is no disinfection apparatus for drainage piping, the bacteria and viruses can be prevented from leaking into the external environment by treating the wastewater from the drainage pipe with only the infectious waste treatment apparatus 301.

Next, the drainage pipe and the raw water tanks will be explained.

Infectious wastewater is discharged to the raw water tanks 302 of the wastewater sterilization treatment apparatus through single wastewater piping 4, 204, 304 so as not to mix with other wastewater.

In cases where "biofilms" serving as places for proliferation of viruses and bacteria are generated inside the drainage pipe 4, 204, 304, the biofilms are decomposed and removed by chlorine dioxide, and hence, there is no fear of secondary contamination due to the backflow of air flow in the piping caused by the loss of water seal in the traps of the toilet bowl 3, the sanitary appliance 20 and the like. Therefore, there is no fear that pathogens, viruses, or the like will flow back into infectious disease rooms from the dry piping by riding on the air flow and causing secondary contamination. In addition, if drainage pipe 4, 204, 304 is connected to drainage piping other than the infectious system, there is a possibility of secondary contamination in the rooms of departments other than the infectious system through this drainage piping other than the infectious system. By providing the dedicated drainage piping in this way, it is possible to prevent secondary contamination in the rooms of departments other than the infectious system.

On the other hand, in the disinfection apparatus for the drainage pipe 4 of the toilet bowl 3, when the sensor 8 senses the supply of water to the cleansing tank 2, a fixed amount of chlorine dioxide is injected into the cleansing tank 2 by means of the chemical liquid pump 9. For example, in cases where cleansing water flows approximately 12 [liters] at a time, chlorine dioxide is injected so that the chlorine dioxide concentration becomes 100 ppm.

Assuming 10 uses per day, approximately 120 liters of chlorine dioxide water will be used to cleanse the drainage pipe.

Although the screens 321 require regular maintenance, a favorable environment is maintained by always cleansing them with a predetermined concentration, e.g., 100 ppm of chlorine dioxide water.

Further, pathogens and viruses are always present in the raw water tanks 302 due to infectious wastewater and contaminants, and there is a risk of proliferation thereof, but the raw water tanks 302 are filled with the predetermined concentration of chlorine dioxide water that flows in through the screens 321 from the drainage pipe 304, so that an inactivated environment is maintained.

In this embodiment, two raw water tanks 302 are provided. The raw water tank 302 into which wastewater flows may be any one of them, and a means is provided for switching to another raw water tank 302 when the amount of wastewater stored in the raw water tank 302 in use exceeds an acceptable amount. The means of switching is configured as follows. That is, the drainage pipe 304 is branched into two branch pipes 304a, 304b before the raw water tanks 302, and the branch pipes 304a, 304b are connected to the raw water tanks 302, 302 via the screens 321, 321, respectively. The two raw water tanks 302, 302 are used alternately, and switching valves 322, 322 are arranged in the branch pipes 304a, 304b, respectively.

For example, in cases where the raw water tank 302 on the side of the branch pipe 304a is used, one switching valve 322 opens the flow passage of one branch pipe 304a, and the other closes the flow passage of the other branch pipe 304b. When the liquid level in the one raw water tank 302 reaches a predetermined height, the switching valves 322 are switched in such a manner that the one branch pipe 304a is closed, and the other branch pipe 304b is opened.

The liquid level in each raw water tank 302 is detected by using a known liquid level detector provided in each raw water tank 302, so that, based on detection signals therefrom, the switching valves 322, 322 such as electromagnetic valves or the like are operated by an unillustrated control unit to switch the flow passages. The control unit may be constituted by a control panel of the entire infectious waste treatment apparatus 301.

For the supply from each raw water tank 302 to the reaction tank 303, water is supplied from a raw water tank 302 in use by the pump of a corresponding wastewater supply device 305.

The supply of wastewater from the raw water tanks 302 to the reaction tank 303 is configured such that in cases where wastewater is supplied from a raw water tank 302 into which wastewater is flowing, the supply of wastewater from the raw water tank 302 to the reaction tank 303 is continued until the amount of wastewater stored in the raw water tank becomes equal to or less than a specified amount, even after the amount of wastewater stored therein exceeds the acceptable amount to switch the supply from the raw water tank 302 to the other raw water tank 302, whereas when the amount of stored wastewater becomes equal to or less than the specified amount, the supply of wastewater to the reaction tank 303 is switched from the raw water tank 302 to the other raw water tank 302. That is, even if one raw water tank 302 in use becomes full and is switched to the other raw water tank 302, wastewater is supplied from the one raw water tank 302 until the one raw water tank 302 becomes empty, i.e., until the liquid level therein drops to a predetermined height, whereas when the liquid level reaches a lower limit value, the wastewater supply device 305 for this raw water tank 302 is stopped, and the wastewater supply device 305 of the other raw water tank 302 is driven to pump up wastewater and supply it to the reaction tank 302. Whether or not the lower limit value of the liquid level is reached can be detected by a known liquid level detector. Based on this detection signal, the driving of each of the wastewater supply devices 305, 305 is switched. This control of the wastewater supply devices 305,305 is also performed by the control unit of the infectious waste treatment apparatus 301.

In the treatment in the reaction tank 303, the fixed amount of wastewater is supplied thereto from the raw water tanks 302, treated therein for a predetermined period of time, and then discharged therefrom, as a result of which the raw water tanks 302 serve as a buffer between the volume of wastewater to be treated and the volume of wastewater to be discharged into the raw water tanks.

If two raw water tanks 302 are provided as in the illustrated example, there will be an advantage in that the facility can be reduced in size, and maintenance, such as cleaning of the raw water tanks 302 or the like, can be performed without stopping the treatment facility by stopping the raw water tank 302 to be maintained while keeping the other raw water tank 302 in operation. Here, note that in this example, the case of two raw water tanks 302 is exemplified, but there may be three or more.

Note that supply paths 324a, 324b from the respective raw water tanks 302, 302 to the reaction tank 303 merge into common piping 324 and are connected therethrough to the reaction tank 303, and in the individual supply paths 324a, 324b, there are arranged electrically operated switching valves 323, 323 such as solenoid valves or the like, respectively. These switching valves 323, 323 are operatively connected with the wastewater supply devices (pumps) 305, respectively, which supply wastewater to the reaction tank 303. Since wastewater is supplied to the reaction tank 303 from either one of the raw water tanks 302, the flow passage of one supply path on the side of the wastewater supply device 305 which is in operation, among the supply paths 324a, 324b on the supply side, is opened, and the flow passage of the other supply path is closed. In this way, it is possible to prevent wastewater from flowing into the other raw water tank 302.

### Derived Form

In addition to infectious disease rooms and nursing care facilities, for the same purpose, a piping disinfection apparatus may be arranged in sanitary equipment in those sections of an automatic cleansing and sterilization system in which infectious wastewater is generated, so that the infectious wastewater pipes from various locations can be effectively cleansed and sterilized.

### DESCRIPTION OF REFERENCE SIGNS

1 disinfection apparatus
2 cleansing tank
3 toilet bowl
4 drainage pipe
5 replenishment water piping
6 water supply system
7 chemical liquid tank
8 sensor
9 chemical liquid pump
10 control unit
11 chemical liquid piping
12 float valve
12a float
17 control wiring
201 disinfection apparatus
202 cleansing tank
203 sanitary equipment
203a hand wash basin
203b waste sink
204 drainage pipe
205 replenishment piping
206 water supply system
206a branch piping
207 chemical liquid tank
208 sensor
209 chemical liquid pump
210 control unit
211 chemical liquid piping
212 float valve
212a float
213 cleansing water piping
213a common piping
213b branch piping
214 electric valve
215 water faucet
216 discharge port
217 control wiring
300 infectious drainage treatment system
301 infectious waste treatment apparatus
302 raw water tank
303 reaction tank
304 drainage pipe, 304a, 304b branch piping
305 wastewater supply device
306 fine bubble generating device
307 circulation path
308 circulation device
309 chemical liquid injection device
309a ozone supplying device
310 discharge tank
311 discharge device
321 screen
322 switching valve
323 switching valve

## Claims

1. An infectious drainage treatment system comprising a disinfection apparatus for drainage piping of sanitary equipment, and a wastewater sterilization treatment apparatus arranged downstream of the drainage piping;
wherein the disinfection apparatus for drainage piping of sanitary equipment comprises a cleansing tank, a toilet bowl which serves as the sanitary equipment, and a drainage pipe connected to the toilet bowl, wherein when the sanitary equipment is used, a fixed amount of cleansing water is supplied to the toilet bowl from the cleansing tank, and the cleansing water after cleansing the toilet bowl is drained from the drainage pipe;
wherein the disinfection apparatus for drainage piping of sanitary equipment further comprises:
replenishment piping configured to replenish, from a water supply system, a fixed amount of water corresponding to the amount of cleansing water supplied from the cleansing tank to the toilet bowl;
a sensor configured to sense the supply of water to the cleansing tank;
a chemical liquid tank configured to store a fixed concentration of chlorine dioxide;
a chemical liquid pump configured to supply a fixed amount of chemical liquid from the chemical liquid tank to the cleansing tank; and
a control unit configured to operate the chemical liquid pump by means of a water supply sensing signal from the sensor to supply a fixed amount of chlorine dioxide to the cleansing tank, thereby maintaining the concentration of chlorine dioxide in the cleansing water within the cleansing tank at a fixed concentration;
wherein the wastewater sterilization treatment apparatus is a treatment apparatus that performs treatment with chlorine dioxide and treatment with ozone on wastewater from the drainage pipe; and
wherein the wastewater sterilization treatment apparatus has a raw water tank for storing wastewater from the drainage pipe, and the drainage pipe is connected to a screen for removing foreign substances flowing into the raw water tank.

2. An infectious drainage treatment system comprising a disinfection apparatus for drainage piping of sanitary equipment, and a wastewater sterilization treatment apparatus arranged downstream of the drainage piping;
wherein the disinfection apparatus for drainage piping of sanitary equipment comprises sanitary equipment with a water faucet for water supply, and a drainage pipe connected to the sanitary equipment;
wherein the disinfection apparatus for drainage piping of sanitary equipment, in which wastewater is drained from the drainage pipe, further comprises:
a cleansing tank configured to store cleansing water for cleansing the sanitary equipment;
a discharge port for the cleansing water provided in the sanitary equipment separately from the water faucet;
cleansing water piping connecting between the cleansing tank and the discharge port;
an electric valve provided in the cleansing water piping to open and close a flow passage in the cleansing water piping;
replenishment piping configured to replenish, from a water supply system, a fixed amount of water corresponding to the amount of cleansing water supplied from the cleansing tank to the sanitary equipment;
a sensor configured to sense the supply of water to the cleansing tank;
a chemical liquid tank configured to store a fixed concentration of chlorine dioxide;
a chemical liquid pump configured to supply a fixed amount of chemical liquid from the chemical liquid tank to the cleansing tank; and
a control unit configured to operate the electric valve at a fixed cycle to supply a fixed amount of cleansing water to the sanitary equipment through the discharge port, and to operate the chemical liquid pump to supply a fixed amount of chlorine dioxide to the cleansing tank by means of a water supply sensing signal from the sensor to maintain the concentration of chlorine dioxide in the cleansing water within the cleansing tank at a fixed concentration;
wherein the wastewater sterilization treatment apparatus is a treatment apparatus that performs treatment with chlorine dioxide and treatment with ozone on wastewater from the drainage pipe; and
wherein the wastewater sterilization treatment apparatus has a raw water tank for storing wastewater from the drainage pipe, and the drainage pipe is connected to a screen for removing foreign substances flowing into the raw water tank.

3. An infectious drainage treatment system comprising a disinfection apparatus for first drainage piping of first sanitary equipment, a disinfection apparatus for second drainage piping of second sanitary equipment, and a wastewater sterilization treatment apparatus arranged downstream of the first drainage piping and the second drainage piping;
wherein the disinfection apparatus for first drainage piping of first sanitary equipment comprises a first cleansing tank, a toilet bowl which serves as the first sanitary equipment, and a first drainage pipe connected to the toilet bowl, wherein when the toilet bowl is used, a fixed amount of cleansing water is supplied to the toilet bowl from the first cleansing tank, and the cleansing water after cleansing the toilet bowl is drained from the first drainage pipe;
wherein the disinfection apparatus for first drainage piping of first sanitary equipment further comprises:
replenishment piping configured to replenish, from a water supply system, a fixed amount of water corresponding to the amount of cleansing water supplied from the first cleansing tank to the toilet bowl;
a sensor configured to sense the supply of water to the first cleansing tank;
a chemical liquid tank configured to store a fixed concentration of chlorine dioxide;
a chemical liquid pump configured to supply a fixed amount of chemical liquid from the chemical liquid tank to the first cleansing tank; and
a control unit configured to operate the chemical liquid pump by means of a water supply sensing signal from the sensor to supply a fixed amount of chlorine dioxide to the first cleansing tank, thereby maintaining the concentration of chlorine dioxide in the cleansing water within the first cleansing tank at a fixed concentration;
wherein the disinfection apparatus for second drainage piping of second sanitary equipment comprises second sanitary equipment with a water faucet for water supply, and a second drainage pipe connected to the second sanitary equipment;
wherein the disinfection apparatus for second drainage piping of sanitary equipment, in which wastewater is drained from the second drainage pipe, further comprises:
a second cleansing tank configured to store cleansing water for cleansing the second sanitary equipment;
a discharge port for the cleansing water provided in the second sanitary equipment separately from the water faucet;
cleansing water piping connecting between the cleansing tank and the discharge port;
an electric valve provided in the cleansing water piping to open and close a flow passage in the cleansing water piping;
replenishment piping configured to replenish, from a water supply system, a fixed amount of water corresponding to the amount of cleansing water supplied from the cleansing tank to the second sanitary equipment;
a sensor configured to sense the supply of water to the second cleansing tank;
a chemical liquid tank configured to store a fixed concentration of chlorine dioxide;
a chemical liquid pump configured to supply a fixed amount of chemical liquid from the chemical liquid tank to the cleansing tank; and
a control unit configured to operate the electric valve at a fixed cycle to supply a fixed amount of cleansing water to the second sanitary equipment through the discharge port, and to operate the chemical liquid pump to supply a fixed amount of chlorine dioxide to the second cleansing tank by means of a water supply sensing signal from the sensor to maintain the concentration of chlorine dioxide in the cleansing water within the second cleansing tank at a fixed concentration;
wherein the wastewater sterilization treatment apparatus is a treatment apparatus that performs treatment with chlorine dioxide and treatment with ozone on wastewater from the first drainage pipe and the second drainage pipe; and
wherein the wastewater sterilization treatment apparatus has a raw water tank for storing wastewater from the first drainage pipe and the second drainage pipe, and the drainage pipes are connected to a screen for removing foreign substances flowing into the raw water tank.

4. The infectious drainage treatment system according to claim 1 or 2, wherein the drainage pipe is a dedicated drainage pipe.

5. The infectious drainage treatment system according to claim 3, wherein the first drainage pipe and the second drainage pipe are dedicated drainage pipes.

6. The infectious drainage treatment system according to any one of claims 1 through 5, wherein a plurality of raw water tanks are provided, and the raw water tank into which wastewater flows is any one of them, and a means is provided for switching to another raw water tank when an amount of wastewater stored in a raw water tank in use exceeds an acceptable amount.

7. The infectious drainage treatment system according to claim 6,
wherein the wastewater sterilization treatment apparatus is configured to supply a fixed amount of wastewater from the raw water tanks to a reaction tank for treatment; and
wherein the supply of wastewater from the raw water tanks to the reaction tank is done in such a manner that in cases where wastewater is supplied from a raw water tank into which wastewater is flowing, the supply of wastewater from the raw water tank to the reaction tank is continued until the amount of wastewater stored in the raw water tank becomes equal to or less than a specified amount, even after the amount of wastewater stored therein exceeds the acceptable amount to switch the supply from the raw water tank to another raw water tank, whereas when the amount of stored wastewater becomes equal to or less than the specified amount, the supply is switched to the supply from the other raw water tank.

8. The infectious drainage treatment system according to any one of claims 1 through 6, wherein a reaction tank of the wastewater sterilization treatment apparatus is provided with a circulation path that serves to discharge treated wastewater to the outside and then return it thereto, and a fine bubble supplying means is provided for supplying ozone to the circulation path as fine bubbles.

9. An infectious drainage treatment system **characterized by** comprising a wastewater sterilization treatment apparatus arranged downstream of drainage piping;
wherein the wastewater sterilization treatment apparatus performs treatment with chlorine dioxide and treatment with ozone on wastewater from the drainage piping; and
wherein a reaction tank of the wastewater sterilization treatment apparatus is provided with a circulation path that serves to discharge treated wastewater to the outside and then return it thereto, and a fine bubble supplying means is provided for supplying ozone to the circulation path as fine bubbles.

10. The infectious drainage treatment system according to claim 9, wherein the chlorine dioxide is also supplied into wastewater as fine bubbles through a fine bubble generating device.
